# FASCICULE DE BREVET EUROPEEN

(11) **EP 0 797 681 B1**
(45) Date de publication et mention de la délivrance du brevet: **23.09.1998**
(21) Numéro de dépôt: 96901377.0
(22) Date de dépôt: 11.01.1996
(51) Int. Cl.: C12P 7/26, C12P 7/22

(54) **PROCEDE DE PREPARATION DE DERIVES DE LA BUTANONE**
VERFAHREN ZUR HERSTELLUNG VON BUTANONDERIVATEN
METHOD FOR PREPARING BUTANONE DERIVATIVES

(30) Priorité: 12.01.1995 FR 9500472
(43) Date de publication de la demande: 01.10.1997
(73) Titulaire: ROBERTET S.A., 06333 Grasse Cédex (FR)
(72) Inventeur: JOULAIN, Daniel, 06130 Grasse (FR); FUGANTI, Claudio, 20129 Milano (IT)
(74) Mandataire: Rinuy, Santarelli
(86) Numéro de dépôt international: FR9600045
(87) Numéro de publication internationale: WO9621739

(56) Documents cités:
- EP-A- 0 186 365
- AGRICULTURAL AND BIOLOGICAL CHEMISTRY, vol. 48, no. 6, Juin 1984 TOKYO, pages 1509-1516, HIROMICHI OHTA ET AL

## Description

La présente invention concerne un procédé enzymatique de préparation de dérivés aromatiques de la butanone.

Il existe un intérêt général confirmé pour produire dans des conditions physiologiques des produits naturels rares, et en particulier ceux qui possèdent des propriétés organoleptiques intenses. Ces procédés sont d'autant plus attractifs lorsqu'ils peuvent être réalisés à partir de précurseurs naturels aisément disponibles. Les produits obtenus de cette manière étant considérés comme naturels, ils peuvent entrer dans la composition d'arômes naturels, lesquels ont la préférence des consommateurs.

Dans cette catégorie de substances, on peut citer des dérivés arylés de la butanone et notamment des dérivés de la phénylbutanone tels que la "cétone framboise", la "zingérone" et la "Cassione ®" (marque déposée par la société Firmenich).

La cétone framboise ou 4-(4-hydroxyphényl) -butan-2-one, a été décrite pour la première fois comme constituant caractéristique de l'arôme de framboise par H. Schinz et C.F Seidel, Helv. Chim. Acta, 1957, 40, 1839. Toutefois, cette substance était déjà connue à l'état naturel comme constituant, entre autres, d'une polygonacée : Rheum palmatum, tant sous la forme d'aglycone, que sous la forme de glucosides (T. Murakami et coll., Tetrahedron Letters, 1972, 2965 ; Y.Kashiwada et coll., Chem. Pharm. Bull., 1986, 34, 3237) . Un gallylglucoside de la cétone framboise a aussi été identifié dans une crassulacée : Aeonium lindleyi (A.G. Gonzalez et coll., Phytochemistry, 1976, 15, 344). Selon une étude récente, le glucoside de la cétone framboise accompagne l'aglycone dans la framboise (A.Pabst et coll. Phytochemistry, 1990, 29, 3853).

La "Cassione ®", ou 4-(3,4-méthylènedioxyphényl)-butan-2-one, n'avait pas été trouvée dans la nature jusqu'à une date récente. Pour cette raison, en dehors de la parfumerie, son emploi n'était autorisé que dans les arômes alimentaires artificiels seulement. On a établi récemment sa présence dans l'huile essentielle de la partie aérienne d'une rue : Ruta angustifolia Pers., plante qui croît à l'état spontané en Malaisie (D. Joulain et Coll. Journal of Essential Oil Research, 1991, 3, 355), et il est donc possible d'utiliser maintenant cette substance dans des arômes alimentaires libellés "naturels".

La zingérone est un constituant du gingembre et de l'arôme de la framboise, mais sa contribution dans cet arôme est nettement moins déterminante que celle de la cétone framboise. Ainsi la proportion de cétone framboise dans la framboise n'est que de 9 à 174 µg seulement par kilogramme de fruits frais, suivant les variétés (W.Borejsza-Wysocki et coll., J.agric. Food Chem., 1992, 40, 1176).

La valeur aromatique de la cétone framboise est exprimée par son seuil de détection olfactive dans l'eau, compris entre 1 et 10 parties par milliard (M. Larsen et coll., Z.Lebensm. Unters. Forsch. 1990, 191, 129)

On comprend donc que la cétone framboise est une substance absolument nécessaire, quoique non suffisante pour réaliser un arôme de framboise reconstitué.

La cétone framboise est aisément disponible sur le marché comme produit synthétique à un coût très modéré, et on l'utilise donc couramment dans des compositions d'arômes identiques à la nature ou artificiels.

Par contre, il n'existe pas de source pratique de cétone framboise naturelle isolée, indispensable pour reconstituer un arôme de framboise totalement naturel. Les concentrations dans lesquelles existe cette substance dans la framboise-fruit sont en effet si faibles, même dans le meilleur des cas, que tout procédé d'extraction en vue d'isoler cette substance serait sans intérêt sur le plan économique. Une situation analogue, quoique à un degré moindre, existe pour la Cassione ® , en raison de sa faible teneur dans l'huile essentielle de rue et de la grande difficulté pour l'isoler avec une pureté suffisante.

Les tentatives pour produire de la cétone framboise par fermentation de novo au moyen de certaines souches de Nidularia n'ont permis de produire que des concentrations de cétone framboise du même ordre de grandeur que celles qui sont observées dans le fruit (P. Tiefel et R.G. Berger dans Progress in Flavour Precursor Studies, P. Schreier et P. Winterhalter, éditeurs, Allured Publishing Co., Carol Stream, III, 1993, P 439-450). Lorsque ces mêmes auteurs ajoutent dans le milieu de culture un précurseur supposé de la cétone framboise comme l'acide paracoumarique, ils observent bien une augmentation de la concentration de la cétone framboise, mais à un niveau qui reste toutefois excessivement faible. La complexité du mélange de bioconversion et les longues durées d'incubation requises sont également des éléments défavorables. Enfin, la toxicité vis à vis du microorganisme des diverses substances phénoliques considérés comme précurseurs possibles de la cétone framboise (ainsi que la cétone framboise elle-même), constitue un problème sévère de levée d'inhibition. Par conséquent, tous ces éléments indiquent qu'un tel procédé est inadapté pour la production à l'échelle préparative de la cétone framboise.

Il serait donc souhaitable de disposer d'un procédé efficace et rentable de préparation, la plus naturelle possible, de dérivés du type arylbutanone.

Or la demanderesse a découvert à présent un procédé de préparation d'un dérivé de la butanone de formule (I) 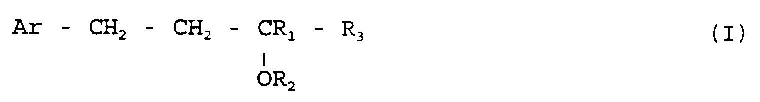 dans laquelle Ar représente un groupement aryle éventuellement mono ou plurisubstitué par des radicaux de préférence alkyle en C₁-C₆, alkoxy en C₁-C₆, méthylènedioxy, ou hydroxyle, R₁ représente un atome d'hydrogène ou représente ensemble avec R₂ une liaison carbone-oxygène, R₂ représente un atome d'hydrogène ou R₂ et R₁ représentent ensemble une liaison carbone-oxygène, et R₃ représente un radical alkyle en C₁-C₆, de préférence un radical méthyle, caractérisé en ce que l'on soumet un composé de formule (II) 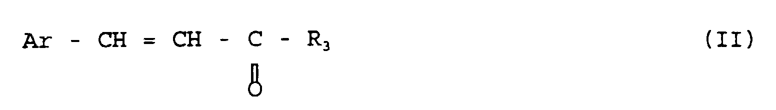 dans laquelle Ar et R₃ ont la signification déjà indiquée, à l'action d'enzymes d'une levure ou d'un champignon filamenteux pour obtenir un mélange de composés de formule I dans laquelle Ar, R₁, R₂ et R₃ ont la signification déjà indiquée, que l'on isole et sépare si désiré.

Il est bien connu aujourd'hui que la réduction d'un dérivé carbonylé α,β-éthylénique par la levure de boulanger procède par réduction totale de la fonction éthylènique, conduisant essentiellement à un alcool saturé. Fogliato et coll. (Tetrahedron 1995, 51(37), page 10231) ont montré que la réduction d'α-arylidènecyclanones par la même levure procéde par l'intervention de différentes enzymes pour conduire à un mélange de dérivé carbonylé saturé, et de (S) - carbinol allylique résultant de la réduction du seul groupe carbonyle, la proportion de ce dernier variant de 35 à 50%. Selon la présente demande, les α-arylidènecétones de formule (II) ont, de façon imprévisible, un comportement très différent. En effet, lorsqu'on les soumet à l'action de différents microoganismes suivant l'invention, incluant même la levure de boulanger, on observe la formation prédominante de la cétone saturée, accompagnée seulement de quantités très mineures de carbinol saturé, et surtout la totale absence de (S)-carbinol allylique.

Dans la formule (I) et dans ce qui suit, par "groupement aryle" on entend un groupement dérivé d'un noyau par substitution d'un atome d'hydrogène, ledit noyau renfermant de 6 à 10 atomes de carbone et comprenant éventuellement un ou plusieurs hétéroatomes, de préférence l'oxygène, tel qu'un radical phényle ou naphtyle. Lorsque le groupement aryle comporte un ou plusieurs hétéroatomes, il comporte de préférence deux et plus particulièrement un seul hétéroatome. Ledit groupement aryle est de préférence au moins monosubstitué. Il comporte de préférence au plus quatre substitutions, et notamment au plus deux substitutions. On peut citer comme substituants préférés par exemple les radicaux méthyle, méthoxy, éthoxy, méthylènedioxy ou hydroxyle.

Dans ce qui suit, les levures et les champignons filamenteux seront désignés ensemble "microorganismes".

Parmi les microorganismes utilisables selon l'invention, on retient notamment pour les levures le genre Saccharomyces, notamment l'espèce delbueckii (CBS 1146) et tout particulièrement l'espèce cerevisiae; on retient encore le genre Hansenula, et notamment l'espèce anomala (CBS 110), ainsi que le genre Debariomices, et notamment l'espèce hansenii (CBS 116). On retient notamment pour les champignons filamenteux ceux du genre Pichia, particulièrement les espèces ohmeris (CBS 5367), stipitis (CBS 5773) et tout particulièrement etchellsii (CBS 2011).

L'action d'enzymes de la levure de boulanger peut être mise en oeuvre selon plusieurs méthodes. Dans une méthode particulièrement préférée, on utilise les microorganismes en fermentation.

En effet, une telle mise en oeuvre permet la préparation des produits attendus avec un excellent rendement et à un très faible coûts des produits mis en oeuvre.

Des conditions avantageuses de fermentation dans le cas de la levure de boulanger (Saccharomyces cerevisiae) sont par exemple les suivantes : On ajoute 1 partie en poids de glucose (ou une quantité équivalente de sucrose ou de mélasse de sucre de betterave, etc) à une suspension agitée de 10 parties en poids de levure de boulanger humide, ou la quantité équivalente de levure commercialisée sous forme déshydratée dans 50 parties en poids d'eau courante, à une température de 30 à 35°. Lorsque la fermentation a commencé, on introduit rapidement le précurseur (environ 0,1 partie), et on maintient l'agitation (en laissant la température revenir à l'ambiante) pendant plusieurs jours, par exemple 5 jours. Le milieu réactionnel dans lequel prédomine la biomasse est ensuite traité de façon conventionnelle pour en extraire les produits de la réaction désirés.

C'est pourquoi la présente demande a notamment pour objet un procédé de préparation d'un dérivé de la butanone tel que défini ci-dessus, caractérisé en ce que l'on utilise une levure ou un champignon filamenteux en fermentation, notamment en phase exponentielle de croissance. On obtient une bonne réduction sélective de la cétone de formule II, et on opère ainsi avec un faible ratio biomasse/substrat, qui facilite l'extraction et la purification du produit de formule I attendu.

Par exemple lorsque l'on ajoute de la 4(4-hydroxy-phényl)-but-3-èn-2-one à raison de 1 gramme par litre de milieu, à une culture du champignon filamenteux Mucor subtilissimus (CBS 735.70) en phase de croissance, on observe la disparition totale de celle-ci au bout de 18 heures. Après 14 heures d'incubation, la proportion de produit attendu (cétone framboise) est déjà de 87,9%. Lorsque l'on augmente la durée de l'incubation jusqu'à 24 heures et au-delà, la proportion de la cétone framboise décroît rapidement en faveur de composés de formule I dans laquelle R₁ et R₂ représentent l'hydrogène. Diverses levures conduisent à des résultats similaires.

Par exemple, dans des conditions de culture et d'incubation analogues, Pichia ohmeris (CBS 5367) et Pichia stipitis (CBS 5773) transforment toutes deux 70% du précurseur butèn-2-one de formule II en cétone framboise de formule I, en 24 heures ; après 48 heures, ces levures ne laissent que 13% et 18% respectivement de précurseur inchangé, sans formation de composés de formule I dans laquelle R₁ et R₂ représentent l'hydrogène. Il en va de même pour les levures Hansenula anomala (CBS 110), Saccharomyces delbueckii (CBS 1146) et Debariomices hansenii (CBS 116) bien que celles-ci procurent un composé de formule I dans laquelle R₁ et R₂ représentent l'hydrogène en proportions mineures. Les performances comparées au bout de 48 heures d'incubation de l'ensemble de ces cinq levures sont consignées dans le tableau I.

| LEVURE | % CETONE FRAMBOISE DE FORMULE (I) |
|---|---|
| Pichia ohmeris | 87 |
| Pichia stipitis | 82 |
| Hansenula anomala | 82 |
| Saccharomyces delbueckii | 85 |
| Debariomices hansenii | 90 |

Dans d'autres conditions de mise en oeuvre du procédé selon l'invention, l'on utilise un concentré d'enzymes de levure ou de champignon filamenteux. Un tel concentré est par exemple celui de levure de boulanger commercialisé par la Société SIGMA sous la référence Y-2875.

C'est pourquoi la présente demande a également pour objet un procédé de préparation d'un dérivé de la butanone tel que défini ci-dessus, caractérisé en ce que l'on utilise un concentré d'enzymes de levure ou d'un champignon filamenteux.

Dans encore d'autres conditions de mise en oeuvre du procédé ci-dessus décrit, l'on utilise à titre de concentré d'enzymes de levure ou d'un champignon filamenteux un extrait protéinique . Un tel extrait est par exemple pour la levure de boulanger celui commercialisé par la Société SIGMA sous le nom "enzyme Sigma type 2".

C'est pourquoi la présente demande a également pour objet un procédé de préparation d'un dérivé de la butanone tel que défini ci-dessus, caractérisé en ce que le concentré d'enzymes ci-dessus est un extrait protéinique de levure.

Dans le cas de l'utilisation d'un concentré d'enzymes ou d'extrait, on utilise avantageusement en outre du β-nicotinamide adénine dinucléotide sous sa forme réduite, ou le phosphate correspondant (NADH et NADPH respectivement) . De tels produits sont commercialisés notamment par la Société SIGMA.

Dans le cas d'une mise en oeuvre du procédé ci-dessus en conditions de fermentation, à l'issue de la période d'incubation jugée nécessaire et suffisante pour isoler et purifier le produit de formule I attendu, le pH du milieu est ajusté à 4-5, par exemple par adjonction d'acide citrique, puis on procède simplement à une extraction au moyen d'un solvant non miscible à l'eau, comme le dichlorométhane, le tertiobutyléther, le butanol, l'acétate d'éthyle ou des combinaisons de ces solvants avec des solvants moins polaires comme le cyclohexane. Après évaporation du solvant d'extraction, le résidu est traité de manière à cristalliser le produit de formule I tel la cétone framboise, par exemple au moyen de mélanges acétate d'éthyle/hexane ou éthanol/eau. On obtient alors un produit pur, tel la cétone framboise pure sous la forme de cristaux blancs possédant un point de fusion de 80-81°C.

Lors de la mise en oeuvre du procédé selon l'invention, on prépare de préférence des dérivés de formule (I) dans lesquels Ar représente un radical phényle éventuellement substitué.

C'est pourquoi la présente demande a également pour objet un procédé de préparation d'un dérivé de la butanone tel que défini ci-dessus, caractérisé en ce que Ar représente un radical phényle éventuellement substitué.

Lorsque le radical phényle est substitué, les substituants sont avantageusement situés en méta et/ou para de la chaîne dérivée de la butanone; lesdits substituants sont tout particulièrement choisis parmi les groupements ci-dessus décrits et notamment les radicaux méthoxy, éthoxy, méthylènedioxy et hydroxyle.

C'est pourquoi la présente demande a plus particulièrement pour objet un procédé de préparation d'un dérivé de la butanone tel que défini ci-dessus , caractérisé en ce que le radical phényle est substitué par au moins un groupement choisi parmi les groupements méthoxy, éthoxy, méthylènedioxy et hydroxyle.

Dans des conditions tout à fait préférentielles de mise en oeuvre, l'invention a pour objet un procédé de préparation d'un dérivé de la butanone tel que défini ci-dessus, caractérisé en ce que l'on prépare la 4-(4-hydroxy-phényl)-butan-2-one, la 4-(3,4-méthylènedioxyphényl)-butan-2-one ou la 4-(4-hydroxy-3-méthoxyphényl)-butan-2-one.

L'invention a également plus particulièrement pour objet un procédé de préparation tel que défini ci-dessus, caractérisé en ce que dans la formule (I), R₁ représente ensemble avec R₂ une liaison carbone-oxygène.

Une famille préférée de produits préparés par le procédé selon l'invention répond à la formule 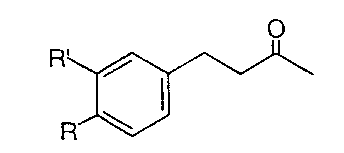 dans laquelle R et R'ont les significations déjà indiquées pour les substituants du groupement aryle.

Dans la mise en oeuvre du procédé ci-dessus, on obtient un mélange de produits de formule (I) dans lequel les dérivés cétoniques sont accompagnés de quantités mineures de l'alcool saturé correspondant.

Selon les dosages chromatographiques effectués durant le déroulement de la réaction, on observe que la double liaison éthylénique est réduite en premier, puis vient le tour de la fonction cétonique. Cette réduction préférentielle d'une double liaison située à la fois en position α d'un groupe carbonyle et conjuguée avec un cycle aromatique est tout à fait inattendue et n'avait jamais été décrite auparavant.

Dans le cas particulier de la préparation de la cétone framboise, correspondant à un dérivé de formule (I) dans lequel Ar représente un radical phényle substitué par un hydroxyle en position 4, on observe que l'alcool secondaire formé par bioréduction de la fonction cétone est sous la configuration S à plus de 85 % comme l'atteste l'analyse du produit brut après acétylation, par chromatographie liquide haute pression (HPLC) en utilisant une colonne chirale (par exemple de type Chiralcel-OD commercialisée par la Société Daicel. Pour cette raison et selon les connaissances antérieures dans ce domaine de la biochimie, il est possible de réoxyder cet alcool secondaire en cétone framboise si désiré, au moyen d'une alcooldéshydrogénase comme par exemple celle extraite du foie de cheval, par exemple en présence d'un excès d'acétaldéhyde comme accepteur d'hydrogène.

Il est possible également d'effectuer la bioréduction autrement qu'avec les cellules entières de microorganismes,notamment de levure en utilisant par exemple un extrait protéinique de ce microorganisme. Dans ce cas, si on n'utilise pas une quantité stoechiométrique de cofacteur, on a avantage à recourir à un système de recyclage de celui-ci (jusqu'à trente fois), qui fait appel à la formiate déshydrogénase. Un tel système d'enzymes est mis en oeuvre de préférence sous une forme immobilisée. Des procédés classiques de l'art antérieur font notamment appel à des polyéthylèneglycols de masse moléculaire de l'ordre de 10.000 et à des membranes permettant le passage de molécules de tailles appropriées.

Les produits de départ de formule (II) sont des produits connus. En particulier, la 4-(4-hydroxyphényl) -but-3-èn-2-one constituant le substrat de réduction en cétone framboise naturelle, peut être obtenue aisément, et sans limite quantitative, par simple contact à chaud entre le 4-hydroxybenzaldéhyde et un excès d'acétone dans une solution aqueuse dont le pH a été amené à une valeur permettant le passage en solution du 4-hydroxybenzaldéhyde. Ces deux éléments sont eux-mêmes disponibles comme substances naturelles. Ainsi l'acétone est obtenue par fermentation du glucose au moyen de Clostridium acetobutylicum notamment. Quant au 4-hydroxy-benzaldéhyde, il peut être libéré par exemple par simple chauffage en présence d'émulsine d'une suspension aqueuse de dhurrine, un glucoside analogue à l'amygdaline qui existe dans les jeunes pousses de sorgo (Sorghum vulgare). Une extraction subséquente au moyen d'un solvant et un lavage pour éliminer l'acide cyanhydrique également libéré dans cette opération sont classiques en pareil cas.

On peut aussi obtenir le 4-hydroxybenzaldéhyde naturel par biodégradation de la picéide, un stibène présent dans l'écorce de l'épicéa, selon Y. Shiotsu et coll., Mukuzai, 1989, 35, page 826.

On voit donc qu'un nombre non négligeable des dérivés de formule (I) peuvent être préparés dans des conditions physiologiques à partir de produits naturels ou issus de produits naturels.

L'invention a enfin pour objet un procédé de préparation d'un dérivé de la butanone tel que défini ci-dessus, caractérisé en ce que en outre, l'on procède à l'oxydation enzymatique usuelle d'un composé de formule (I) dans laquelle R₁ et R₂ représentent un atome d'hydrogène, pour obtenir le composé correspondant de formule (I) dans laquelle R₁ représente ensemble avec R₂ une liaison carbone-oxygène.

Les exemples qui suivent illustrent la présente invention.

### EXEMPLE 1 : Préparation microbiologique de 4-(4-hydroxyphényl)-butan-2-one et de 4-(4-hydroxyphényl)-butan-2-ol.

Dans un récipient à l'air libre, équipé d'un système d'agitation, on introduit successivement 2,5 litres d'eau, puis 100 grammes de D-glucose. Cette solution est ensuite amenée sous agitation à la température de 30°C environ, puis on ajoute en une seule fois 1 kg de levure de boulanger ordinaire sous la forme humide commerciale. La suspension étant vigoureusement agitée à la température de 30°C à 35°C, on observe au bout de quelques minutes le démarrage de la fermentation, visualisé par la formation d'une mousse due à la production de gaz carbonique. On observe aussi une élévation concomitante de la température de 2 à 5°C. La température étant alors voisine de 35°C, on ajoute goutte à goutte en 10 minutes une solution de 8,1 grammes de 4-(4-hydroxyphenyl)-but-3-èn-2-one (0,050 mole) dans 20 ml d'éthanol à 96°C. L'agitation étant maintenue, on laisse revenir à la température ambiante, et on conserve ces conditions pendant 48 heures.

Le milieu d'incubation est alors soumis à une extraction liquide-liquide continue pendant 12 heures au moyen de méthyltertiobutyléther (MTBE). Après élimination du solvant par évaporation, on obtient environ 10 grammes d'un produit brut solide, constitué principalement, d'après analyse par chromatographie en phase gazeuse couplée avec un spectromètre de masse de :
- 56 % de 4-(4-hydroxyphényl)-butan-2-one
- 37 % de 4-(4-hydroxyphényl)-butan-2-ol
- 3 % de substrat de départ inchangé.

On procède alors à la purification du produit de la façon suivante : le produit brut est repris à la température de 60°C environ avec le minimum d'acétate d'éthyle, puis à cette solution limpide de couleur jaune foncé, on ajoute goutte à goutte de l'hexane jusqu'à l'apparition d'un début de turbidité. On abandonne cette solution à la température ambiante. Un produit cristallisé se sépare. On l'essore et on obtient 4,8 g de 4-(4-hydroxyphényl)-butan-2-one pure à plus de 99 %. Une recristallisation dans un mélange d'eau et de méthanol fournit un produit de qualité analytique possédant un point de fusion de 84°C.

### EXEMPLE 2 : Préparation enzymatique de 4-(4-hydroxyphényl)-butan-2-one et de 4-(4-hydroxyphényl)-butan-2-ol.

A une solution de 100 mg d'extrait protéinique de levure, tel que celui qui est commercialisé par la Société Sigma sous la référence Y- 2875 dans 10 ml de tampon phosphate (pH 7), et maintenue agitée à la température de 20°, on ajoute 20 mg de 4-(4-hydroxyphényl)-but-3-èn-2-one et 70 mg de β-nicotinamide adénine dinucléotide sous la forme réduite (NADH), produit commercialisé par la Société Sigma sous la référence N-8129.

Après 24 heures d'agitation à cette température, on effectue une analyse du mélange réactionnel par chromatographie sur couche mince, en observant l'apparition du produit attendu. Après 75 heures, on extrait le mélange réactionnel avec trois fois 2 ml d'acétate d'éthyle. L'analyse du produit brut de réaction par chromatographie en phase gazeuse couplée à un spectromètre de masse indique que celui-ci est constitué essentiellement de substrat de départ, de cétone framboise et de 4-hydroxybenzaldéhyde dans les proportions de 100/5/0,6.

### EXEMPLE 3 : Préparation enzymatique de 4-(4-hydroxyphényl)-butan-2-one et de 4-(4-hydroxyphényl)-butan-2-ol.

A une solution de 50 mg d'extrait d'enzyme Sigma, type 2 et 35 mg de phosphate de β-nicotinamide adénine dinucléotide sous forme réduite (NADPH), commercialisé par la Société Sigma sous la référence N-1630, dans 5 ml de tampon phosphate 0,1 M (pH 7) à la température de 25°C, on ajoute 10 mg de 4-(4-hydroxyphényl)-but-3-èn-2-one. Après 6 heures d'agitation à cette température, on observe que le taux de conversion est de 14 %. On ajoute alors 25 mg d'extrait enzymatique supplémentaires. Le contrôle analytique du déroulement de la bioconversion indique que le taux de conversion atteint un maximum de 30 % au bout de 20 heures environ.

### EXEMPLE 4 : Préparation enzymatique de 4-(4-hydroxyphényl)-butan-2-one à partir de 4-(4-hydroxyphényl)-butan-2-ol.

A une solution 0,050 M d'acide 3-(N-morpholino)propanesulfonique (pH 7) agitée, on ajoute successivement à la température ambiante de 20°C : 1 mg de NAD ⁺ , 2 mg de HLADH (alcool déshydrogénase de foie de cheval), 60 microlitres d'acétaldéhyde et 8,3 mg de 4-(4-hydroxyphényl)-butan-2-ol sous la forme d'un excès énantiomérique S de 64 %. Au bout de 40 heures d'agitation à cette température, on extrait le milieu réactionnel, on l'analyse par chromatographie comme précédemment et on observe la présence de 4-(4-hydroxyphényl)-butan-2-one et de 4-(4-hydroxyphényl) -butan-2-ol que l'on isole. Après acétylation, l'analyse de cet alcool par chromatographie chirospécifique en phase liquide indique qu'il s'agit principalement de l'énantiomère R, avec un excès énantiomérique de 84 %.

### EXEMPLE 5 : Préparation microbiologique de 4-(4-hydroxyphényl)-butan-2-one.

On procède à une culture de Mucor subtilissimus (CBS 735.70), déposé le 22 décembre 1995 à la collection Nationale de Cultures de Microorganismes de l'Institut Pasteur à Paris sous le numéro I-1653, sur un milieu solide (malt: 20 g/l, peptone: 5 g/l, glucose: 20 g/l, agar: 15 g/l), à la température de 24°C pendant 4 jours. Cette culture est utilisé pour inoculer une fiole de 300 ml contenant 50 ml d'un milieu de culture constitué de malt/ 20 g/l, peptone: 5 g/l, glucose: 20 g/l, à raison de 5 ml environ pour 50 ml de milieu. Après 24 heures d'agitation à la température de 24°C, à raison de 140 secousses par minute, cette culture est elle-même utilisée comme inoculum, et introduite dans un fermenteur de 6 litres contenant le même milieu, à raison de 5% par rapport à la quantité de ce milieu. Après 12 à 24 heures de croissance du champignon, on ajoute sous la forme d'une solution dans le minimum d'éthanol à 96% à raison de 1 à 2 gramme par litre de milieu de la 4-(4-hydroxyphényl)-but-3-ène-2-one.

Au bout de 15 heures, l'état d'avancement de la transformation est rapidement constaté par analyse d'une prise d'essai, et on procède à une extraction après avoir ajouté la quantité suffisante d'acide citrique pour amener le pH du milieu à 5. Après traitements habituels, on isole comme dans l'exemple 1 la cétone framboise cristalisée ou 4-(4-hydroxyphényl)-butan-2-one avec un rendement molaire de 80%.

### EXEMPLE 6 : Préparation microbiologique de 4-(4-hydroxyphényl)-butan-2-one.

Un procédé analogue à celui de l'exemple 5 a été mis en oeuvre au départ de la levure Pichia etchellsii (CBS 2011), déposée à la Collection Nationale de Cultures de Microoganismes de l'Institut Pasteur à Paris le 22 décembre 1995 sous le numéro I-1652; la cétone framboise est obtenue également avec un excellent rendement.

## Revendications

1. Procédé de préparation d'un dérivé de la butanone de formule (I) 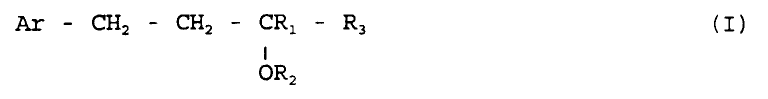 dans laquelle Ar représente un groupement aryle éventuellement mono ou plurisubstitué par des radicaux alkyle en C₁-C₆, alkoxy en C₁-C₆, méthylènedioxy ou hydroxyle, R₁ représente un atome d'hydrogène ou représente ensemble avec R₂ une liaison carbone-oxygène, R₂ représente un atome d'hydrogène ou R₂ et R₁ représentent ensemble une liaison carbone-oxygène,et R₃ représente un radical alkyle en C₁-C₆, caractérisé en ce que l'on soumet un composé de formule (II) 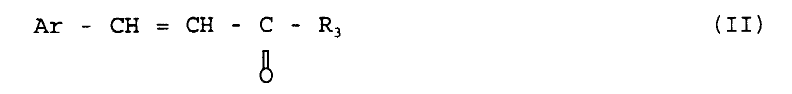 dans laquelle Ar et R₃ ont la signification déjà indiquée, à l'action d'enzymes d'une levure ou d'un champignon filamenteux pour obtenir un mélange de composés de formule I dans laquelle Ar, R₁, R₂ et R₃ ont la signification déjà indiquée que l'on isole et sépare si désiré.

2. Procédé de préparation d'un dérivé de la butanone selon la revendication 1, caractérisé en ce que l'on utilise la levure ou un champignon filamenteux en fermentation.

3. Procédé de préparation d'un dérivé de la butanone selon la revendication 1, caractérisé en ce que l'on utilise un concentré d'enzymes de levure ou d'un champignon filamenteux.

4. Procédé de préparation d'un dérivé de la butanone selon la revendication 3, caractérisé en ce que le concentré d'enzymes de levure ou d'un champignon filamenteux est un extrait protéinique.

5. Procédé de préparation d'un dérivé de la butanone selon l'une des revendications 1 à 4, caractérisé en ce que Ar représente un radical phényle éventuellement substitué.

6. Procédé de préparation d'un dérivé de la butanone selon la revendication 5, caractérisé en ce que le radical phényle est substitué par au moins un groupement choisi parmi les groupements méthoxy, éthoxy, méthylènedioxy et hydroxyle.

7. Procédé de préparation d'un dérivé de la butanone selon l'une des revendications 1 à 6, caractérisé en ce que l'on prépare la 4-(4-hydroxyphényl)-butan-2-one.

8. Procédé de préparation d'un dérivé de la butanone selon l'une des revendications 1 à 6, caractérisé en ce que l'on prépare la 4-(4-hydroxy, 3-méthoxyphényl)-butan-2-one.

9. Procédé de préparation d'un dérivé de la butanone selon l'une des revendications 1 à 6, caractérisé en ce que dans la formule (I), R₁ représente ensemble avec R₂ une liaison carbone-oxygène.

10. Procédé de préparation d'un dérivé de la butanone selon l'une des revendications 1 à 5, caractérisé en ce que en outre, l'on procède à l'oxydation enzymatique usuelle d'un composé de formule (I) dans laquelle R₁ et R₂ représentent un atome d'hydrogène, pour obtenir le composé correspondant de formule (I) dans laquelle R₁ représente ensemble avec R₂ une liaison carbone-oxygène.

## Claims

1. Preparation process for a butanone derivative of formula (I) 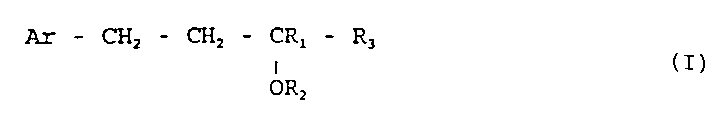 in which Ar represents an aryl group optionally mono- or polysubstituted by C₁-C₆ alkyl, C₁-C₆ alkoxy, methylenedioxy or hydroxyl radicals, R₁ represents a hydrogen atom or represents together with R₂ a carbon-oxygen bond, R₂ represents a hydrogen atom or R₂ and R₁ together represent a carbon-oxygen bond, and R₃ represents a C₁-C₆ alkyl radical, characterised in that a compound with formula (II) 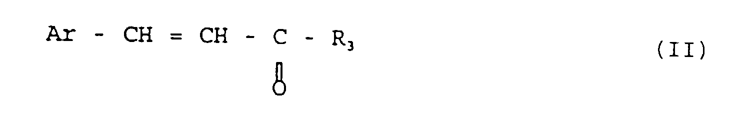 in which Ar and R₃ have the meaning already indicated, is subjected to the action of the enzymes of a yeast or of a filamentous fungus in order to obtain a mixture of compounds of formula I in which Ar, R₁, R₂ and R₃ have the meaning already indicated, which are isolated and separated if desired.

2. Preparation process for a butanone derivative according to claim 1, characterised in that a yeast or a filamentous fungus in fermentation is used.

3. Preparation process for a butanone derivative according to claim 1, characterised in that a concentrate of enzymes of yeast or of a filamentous fungus is used.

4. Preparation process for a butanone derivative according to claim 3, characterised in that the concentrate of enzymes of yeast or of a filamentous fungus is a protein extract.

5. Preparation process for a butanone derivative according to one of claims 1 to 4, characterised in that Ar represents a phenyl radical optionally substituted.

6. Preparation process for a butanone derivative according to claim 5, characterised in that the phenyl radical is substituted by at least one group chosen from the methoxy, ethoxy, methylenedioxy and hydroxyl groupings.

7. Preparation process for a butanone derivative according to one of claims 1 to 6, characterised in that 4-(4-hydroxyphenyl)-butan-2-one is prepared.

8. Preparation process for a butanone derivative according to one of claims 1 to 6, characterised in that 4-(4-hydroxy,3-methoxyphenyl)-butan-2-one is prepared.

9. Preparation process for a butanone derivative according to one of claims 1 to 6, characterised in that in formula (I), R₁ together with R₂ represent a carbon-oxygen bond.

10. Preparation process for a butanone derivative according to one of claims 1 to 5, characterised in that, in addition, the usual enzymatic oxidation of a compound of formula (I) is carried out in which R₁ and R₂ represent a hydrogen atom, in order to obtain the corresponding compound of formula (I) in which R₁ together with R₂ represents a carbon-oxygen bond.

## Patentansprüche

1. Verfahren zur Herstellung eines Butanonderivats der Formel (I) 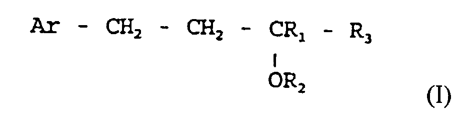 worin Ar eine Arylgruppe ist, gegebenenfalls mono- oder mehrfachsubstituiert mit C₁-C₆-Alkyl-, C₁-C₆-Alkoxy-, Methylendioxy- oder Hydroxylgruppen, R₁ ein Wasserstoffatom oder zusammen mit R₂ eine Kohlenstoff-Sauerstoff-Bindung ist, R₂ ein Wasserstoffatom ist oder R₂ und R₁ zusammen eine Kohlenstoff-Sauerstoff-Bindung sind und R₃ eine C₁-C₆-Alkylgruppe ist, dadurch gekennzeichnet, daß man eine Verbindung der Formel (II) 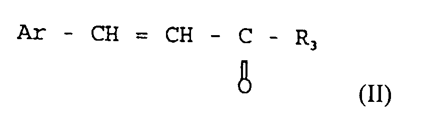 worin Ar und R₃ die bereits angegebenen Bedeutungen haben, der Wirkung von Hefe- oder Faserpilz-Enzymen unterzieht, um eine Mischung von Verbindungen der Formel I zu erhalten, worin Ar, R₁, R₂ und R₃ die bereits angegebenen Bedeutungen haben, die man, wenn gewünscht, isoliert und abtrennt.

2. Verfahren zur Herstellung eines Butanonderivats nach Anspruch 1, dadurch gekennzeichnet. daß man bei der Fermentation die Hefe oder einen Faserpilz verwendet.

3. Verfahren zur Herstellung eines Butanonderivats nach Anspruch 1, dadurch gekennzeichnet, daß ein Konzentrat von Hefe- oder Faserpilz-Enzymen verwendet wird.

4. Verfahren zur Herstellung eines Butanonderivats nach Anspruch 3, dadurch gekennzeichnet, daß das Konzentrat von Hefe- oder Faserpilz-Enzymen ein Proteinextrakt ist.

5. Verfahren zur Herstellung eines Butanonderivats nach einem der Ansprüche 1 bis 4, dadurch gekennzeichnet, daß Ar eine gegebenenfalls substituierte Phenylgruppe darstellt.

6. Verfahren zur Herstellung eines Butanonderivats nach Anspruch 5, dadurch gekennzeichnet. daß die Phenylgruppe mit mindestens einer Gruppe, ausgewählt aus den Gruppen Methoxy, Ethoxy, Methylendioxy und Hydroxyl, substituiert ist.

7. Verfahren zur Herstellung eines Butanonderivats nach einem der Ansprüche 1 bis 6, dadurch gekennzeichnet, daß man 4-(4-Hydroxyphenyl)-butan-2-on herstellt.

8. Verfahren zur Herstellung eines Butanonderivats nach einem der Ansprüche 1 bis 6, dadurch gekennzeichnet, daß man 4-(4-Hydroxy-3-methoxyphenyl)-butan-2-on herstellt.

9. Verfahren zur Herstellung eines Butanonderivats nach einem der Ansprüche 1 bis 6, dadurch gekennzeichnet, daß in der Formel (I) R₁ zusammen mit R₂ eine Kohlenstoff-Sauerstoff-Bindung ist.

10. Verfahren zur Herstellung eines Butanonderivats nach einem der Ansprüche 1 bis 5, femer dadurch gekennzeichnet, daß man eine übliche enzymatische Oxidation einer Verbindung der Formel (I), worin R₁ und R₂ ein Wasserstoffatom sind, durchführt, um die entsprechende Verbindung der Formel (I) zu erhalten, worin R₁ zusammen mit R₂ eine Kohlenstoff-Sauerstoff-Bindung ist.
